Europäisches Patentamt

(19) European Patent Office

Office européen des brevets

(11) Publication number: **0 260 241**
**A1**

(12) # EUROPEAN PATENT APPLICATION

(21) Application number: **87850273.1**

(22) Date of filing: **08.09.87**

(51) Int. Cl.⁴: **A 61 K 9/12**
A 61 K 9/72, A 61 K 47/00,
A 61 K 31/685

(30) Priority: **12.09.86 SE 8603812**

(43) Date of publication of application:
**16.03.88 Bulletin 88/11**

(84) Designated Contracting States: **ES GR**

(71) Applicant: **Aktiebolaget Draco**
**Box 34**
**S-221 00 Lund (SE)**

(72) Inventor: **Axelsson, Bengt Ingemar**
**108 The Oaks Burning Tree Drive**
**Chapel Hill North Carolina 27514 (US)**

**Byström, Ulla Katarina**
**Värnen Kullavägen**
**S-240 13 Genarp (SE)**

**Dahlbäck, Carl Magnus Olof**
**Sven Tveskäggsgränd 14**
**S-223 77 Lund (SE)**

**Källström, Leif Arne**
**Kärrsangarvägen 39**
**S-240 17 Södra Sandby (SE)**

**Nilsson, Per-Gunnar**
**Bertrandsgatan 2A**
**S-212 14 Malmö (SE)**

**Trofast, Jan William**
**Vapenkroken 34**
**S-222 47 Lund (SE)**

(74) Representative: **Hjertman, Ivan T. et al**
**AB ASTRA Patent and Trade Mark Department**
**S-151 85 Södertälje (SE)**

(54) **A new system for administration of liposomes to mammals.**

(57) A new system to administer liposomal formulations to mammals, including man, comprising a dry lipid-based solid material, which in an aqueous milieu spontaneously form or reconstitute liposomes, and a device for aerosolizing a selected quantity of the lipid-based solid material in a suitable form, is described.

The invention is particularily suitable for applications where the lipid-based solid composition is inhaled as a finely divided powder in aerosol form and hydrated in the respiratory tract.

EP 0 260 241 A1

## Description

A new system for administration of liposomes to mammals

Field of invention

The present invention provides a new system to administer liposomal formulations to mammals, including man, comprising a dry lipid-based solid material, which in an aqueous milieu spontaneously form or reconstitute liposomes, and a device for aerosolizing a selected quantity of the dry lipid-based solid material in a suitable form. The invention is particularily concerned with breath-actuated powder-inhalators and self-propelling powder-dispensing compositions capable of dispensing in aerosol form a powder of said dry lipid-based solid materials.

Until now liposomal formulations have been administered to mammals as aqueous dispersions or as compositions consisting of dissolved lipids. It is an object of the present invention to provide a new improved system to administer liposomal formulations. The invention is based on the fact that certain lipid-based solid materials hydrate to form or reconstitute liposomes in an aqueous milieu. The lipid-based solid materials are administered to the body in aerosol from by a suitable device and the hydration to liposomes occurs in vivo, either at the site of action or elsewhere.

Although the aerosolized lipid-based material can be administered to various parts of the body applications where it is inhaled as a finely-divided powder and hydrated in the respiratory tract are particularly suitable. It is therefore a further object of the present invention to provide a pharmaceutical package suitable for inhalation therapy.

Background art

Liposomes

Liposomes are widely described in the literature and their general structure is well known, they are structures composed of concentric rings of lipid bilayers. Liposomes have been used as carriers for different kinds of pharmacologically active substances in order to improve their therapeutic efficacy.

Liposomes can be used as carriers both for hydrophilic and lipophilic substances. Hydrophilic substances are encapsulated in the aqueous space between the lipid bilayers while lipophilic substances are incorporated into the lipid bilayers.

Liposomes can be made from a range of lipids, both neutral and charged, including phophatidylcholines, and can be manufactured in a number of different ways resulting in liposomes with various properties. Liposomes can therefore to some extent be taylored to suit a specific application. Examples of parameters which can be controlled are liposome size (20 nm - µm), type [SUV (small unilamellar vesicles), LUV (large unilamellar vesicles), MLV (multilamellar vesicles) etc], phase-transition temperature, $T_c$ (and hence the rate of release of encapsulated substance) and liposome charge.

One of the major problems in the development of a pharmaceutical liposomal formulation is the long-time stability. Aqueous liposome dispersions have a limited physical stability since the liposomes can aggregate resulting in a change in the size distribution. Furthermore, if the encapsulated drug is hydrophilic it may be lost into the external aqueous phase. In addition, there is a potential risk for chemical degradation of the lipid components and the pharmacologically active substance in an aqueous milieu.

One way to circumvent the physical stability problem is to store the lipid dissoved in an arganic solvent and form liposomes immediately before use. However, also with this approach chemical degradation can be a problem.

The problem concerning stability can to large extent be solved if a dry solid composition is developed. The term "dry" implies a virtually water-free formulation but does not imply the absence of a liquid in which the solid composition is virtually insoluble. A dry solid composition is stable during long time at room temperature and under moisture protection in a closed container.

Two different approaches to provide dry solid compositions have been suggested. The first approach is by liposome precursors, proliposomes. Proliposomes are formulations from which liposomes readily can be prepared, usually by dispersing the proliposome material in an aqueous solution and, if necessary, heating the dispersion to a temperature above the phase-transition temperature of the lipid material (UK Patent Specification 1 575 343, UK Patent Appliccation GB 2 135 268 A).

The second approach to obtain a dry solid composition is to dehydrate a liposome dispersion in the presence of a filler material intended to impede agglomeration of the dehydrated product (UK Patent Application GB 2 002 319 A). The dehydration can for example be performed by lyophilization or by spray-drying. The liposomes are reconstituted by rehydration of the dry product.

The dry solid products of the both approaches discussed above can be used in the present invention. Instead of hydrating the dry solid material before administration as in previous methods, it is in the present invention administered to the body in aerosol form by a suitable device. In the body the dry lipid-based solid material is hydrated to liposomes.

## Inhalation therapy

The administration of pharmacologically active substances by inhalation has been known and employed for many years. For many conditions, especially those which involve allergic and inflammatory diseases in the respiratory tract, inhalation of drugs can be exceedingly useful.

Various inhalation devices such as powder-inhalators (Spinhaler®, Rotahaler® Turbuhaler® etc) and pressurized dose-aerosols exist. A powder-inhalator provides a selected quantity of a dry powder in a form suitable for inhalation. No powder-inhalators containing a lipid-based solid material exist.

The pressurized dose-aerosol is a pressure-tight container having a valve-controlled opening and containing a self-propelling composition capable of providing drug in aerosol form. The self-propelling composition comprises a propellant in which the pharmacologically active substance is dissolved or dispersed. Optionally, adjuvants such as ethanol, nonionic surfactants, taste maskers, etc are added to improve the formulation. On operating the metering valve of the aerosol container, the active material is dispensed in a stream of propellant. Pressurized dose-aerosols containing lipid materials exist on the market. However, these materials are present as suspending agents for the drug particles in the propellant and as valve lubricants.

chemical stability can be a large problem when the lipid material and or the biologically active substances are in a dissolved state.

## Detailed description of the present invention

The present invention provides a new system to administer liposomal formulations to mammals, including man. The system comprises a dry lipid-based solid material and a device for aerosolizing said material. The said material can either be manufactured by dehydration of a liposome dispersion in the presence of a filler material or be a proliposome material. The said materials spontaneously hydrate to form or reconstitute liposomes at the target organ or elsewhere in the body. The device is preferable a breath-actuated powder-inhalator or a pressurized dose-aerosol. The present method is distinguished from previous methods to administer liposomal formulations in that the lipid-based composition is, not dissoved in a solvent or dispersed in water, but in a particulate solid state. The problems concerning chemical stability are therefore eliminated.

The dry lipid-based solid materials used in the present invention contain lipid material and optionaly a pharmacologically active substance. They can also contain at least one adjuvant which imparts advantageous properties.

The lipid material can be any of those conventionally used in liposomal formulations. Usually the main liposome-forming component is a phospholipid, including synthetic lecithins and natural lecithins. In addition to the main liposome-forming component other lipids may be used to optimize the properties of the formulation. Examples of such additives are sterols such as cholesterol or derivatives thereof or components with a positive or negative charge.

Cholesterol or derivatives thereof may be incorporated, preferably in a proportion of 0.1 to 50 % w/w of the total lipids, to modify the membrane structure rendering it more fluid or more rigid and thereby influence the release properties of the entrapped pharmacologically active material. Cholesterol also has a positive effect on the stability of the liposomes during lyophilization.

Components with a negative or positive charge will provide an electrostatic stabilization of the liposomes and may also improve the uptake of the liposomes in the target cells. Examples of negatively charged lipophilic substances are phosphatic acid, dicetyl phosphoric acid, phosphatidyl serine, phosphatidyl inositol and phosphatidyl glycerol. Examples of positively charged lipophilic substances are stearylamine, stearylamine acetate and cetylpyridinium chloride.

Both hydrophilic and lipophilic pharmacologically active substances can be encapsulated into the liposomes. Alternatively, liposomes can be made without any encapsulated substance.

Suitable adjuvants are filler substances such as carbohydrates, for example lactose, sucrose or trehalose. These materials facilate the dispersion of a proliposome material and impede agglomeration of a dehydrated product.

The proliposome formulation used in the present invention can be any which in an aqueous milieu forms liposomes in the temperature range of current interest. Some care must be taken to ensure that liposomes and not other structures are formed when a proliposome material is used.

A material obtained from a dehydrated liposome dispersion gives larger opportunities to taylor the formulation. The liposome dispersion from which the dry solid material is made can be prepared according to any of the numerous methods known in the art. The dehydration can be performed with any method which does not destroy the liposomal structure. Preferably, the dehydration will be carried out by lyophilization or spray-drying in the presence of a filler material.

The system described in the present invention can be used to administer liposomes to various parts of the body. The following routes of administration should be mentioned; oral, rectal, vaginal, in open wounds, and by inhalation. For each application the system must be optimized both with respect to the lipid-based solid composition (components, method of manufacture etc) and the device (breath-actuater powder-inhalator, pressurized aerosol etc).

Inhalation therapy is an attractive application of the present invention. In the respiratory tract there is a large area with high humidity where the lipid-based solid matrial can be hydrated. Various powder-dispensing dose-aerosols which can deliver a powder of the material to the respiratory tract exist.

Pharmacologicaly active substances which are particularly useful for inhalation are $\beta_2$-receptor active substances, xanthines, glucocorticoids, other antiallergic or antiinflammatory substances or derivatives of the substances mentioned.

Suitable $\beta_2$-receptor active substances are terbutaline, salbutamol, mabuterol, fenoterol, formoterol, orciprenaline, isoprenaline, isoetharine, clenbuterol, hexoprenaline, procaterol, 1-(4-hydroxyphenyl)-2-[1,1-dimethyl-3--(2-methoxyphenyl) propylamino]-ethanol, 1-(3,5-dihydroxy-phenyl)-2-[1,1-dimethyl-3-(2-methoxyphenyl) propylamino]--ethanol, 1-3, 4-dihydroxyphenyl-2-[1,1-dimethyl-3-(2--methoxyphenyl) propylamino]-ethanol, (4-hydroxy-$\alpha'$-[[[6 -(4-phenylbutoxy)-hexyl]-amino]-methyl]-1,3 benzyldimethanol, pharmacologically acceptable salts or derivatives thereof and compounds of similar pharmagological properties. The $\beta_2$-receptor active substance which is particularly preferred is terbutaline sulphate.

Among the xanthines theophylline and enprofylline and pharmacologically acceptable salts and derivatives thereof and compounds of similar pharmacological properties are the most suitable.

Suitable glucocorticoids are compounds like budesonide, beclomethasone, dexamethasone, flumethasone, flunisolide, triamcinolone acetonide, 17- and/or 21-esters of these steroids, pharmacologically acceptable salts thereof and compounds of similar pharmacological properties.

Other compounds which can be used in accordance with the present invention are cromoglycate, lidocaine, indometacin, diclofenac, ibuprofen, piroxicam, sulindac, derivatives thereof and compounds of similar pharmacological properties.

Breath-actuated powder-inhalers are suitable devices to administer the lipid-based solid material to the respiratory tract. As said material may be sensitive to moisture it may be necessary to ensure moisture protection. This is also the case for certain other inhalation materials on the market, for example sodium cromoglycate, and can therefore easily be ensured also for said solid-based lipid material.

The pressurized dose-aerosol is for several reasons, which will be discussed below, a particular suitable device. Considerations which must be taken onto account when a pressurized dose-aerosol is developed are therefore discussed below.

The pressurized dose-aerosol can be manufactured according to known technique i.e. a powder of a defined particle-size is suspended in a suitable propellant mixture. The dispersion is filled on containers which are sealed with a metering valve. The water content should be kept as low as possible. The components making up the lipid-based solid composition must be virtually insoluble in the propellant. Further they must of course be chemically stable during storage in the aerosol container. (Since the components are insoluble in the propellant mixture this is generally no problem.) It is often necessary to exclude propellant 11 ($CCl_3F$) from the formulation since it has the highest solvency power among the propellants which normaly are used in pressurized dose-aerosols for medicinal use. By mixing propellant 12 ($CCl_2F_2$) and propellant 114 ($CClF_2CClF_2$) in different proportions pressurized dose-aerosols with the desired pressure can be obtained. If necessary it is possible to minimize the solubility of the solid components by using propellant 115 ($CClF_2CF_3$) or propellant C-318 ($C_4F_8$). These are among the poorest solvents known. Preferably the solid material is suspended in a concentration between 0.001 and 20 % by weight, in the liquified propellant.

The pressurized dose-aerosol can also contain at least one adjuvant which imparts advantageous properties. Such adjuvants may be surfactants, taste-maskers of modifiers of the solubility of the lipid-based solid material in the propellant. Preferred surfactants are those which are soluble in the propellant and consitutes between 0.001 % and 20 % by weight of the total composition.

The solubilities of a range of lipids and pharmacologically active substances in different propellants have been measured. The substance was dispersed in the propellant and the sample was stored in a sealed container under pressure. After equilibrium the sample was filtered and the concentration was determined. Some of the results are presented in Table 1.

Table 1   Solubilities in propellants/$\mu$g x g$^{-1}$

|  | Propellant | | |
|---|---|---|---|
|  | 12 | 114 | 115 |
| budesonide-21-palmitate |  | 170 |  |
| flunisolide-21-palmitate | 60 | 12 |  |
| flumethasone-21-palmitate | 5.9 | 1.0 | < 0.005 |
| flumethasone-21-stearate | 2.4 | 1.5 |  |
| DPPC (dipalmitoyl phosphatidylcholine) |  | < 2 |  |
| DMPC (dimyristoyl phosphatidylcholine) |  | < 2 |  |
| Epikuron$^R$ 200 H (> 95 % phosphatidylcholine, Lucas Meyer, Hamburg) |  | < 2 |  |
| DPPA (dipalmitoyl phosphatic acid) |  | < 10 |  |
| Stearylamine |  | < 10 |  |
| Catalase |  | < 10 |  |

It can be seen from the table that the solubilities of many important substances are very low in the propellants making a suspension aerosol feasible.

The presssurized dose-aerosol described above shows advantages compared to other ways of storing and delivering liposomal formulations. It comprises a package where the material is protected during storage from air, light, moisture etc, which may affect the material. It is easy to administer a dose of the formulation without affecting the rest of the material. The lipid material is in a solid state and therefore there is minimal risk for chemical degradation and for changes in the physical structure. There is no need for expensive aerosol-delivering devices as ordinary aerosol containers and valves can be used (in contrast to technical approaches where special devices are needed to mix the lipid material with an aqueous solution). Finally, the liposomes can be manufacatured to have specific properties (liposome size, drug-loading etc) which may be difficult to obtain when the liposomes ae generated in situ.

Working examples

The present invention is exemplified but in no way limited by the following examples. A variety of other materials and other methods could be used to obtain the desired lipid-based solid composition. Further, other routes and devices could be used to administer the lipid-based solid material. In all examples care was taken to avoid that the dry solid formulation came into contact with moisture.

Example 1

1 g DPPC was dissolved in 50 ml t-butanol under gentle heating. The solution was frozen and lyophilized. The resulting powder was dispersed in 135 g of sterile aqueous solution of lactose (6.67 weight %). Liposomes were formed by heating (maximum temperature 75 °C) the sample for 45 minutes during stirring. The liposome dispersion was flash- frozen by dripping it into liquid nitrogen and was then lyophilized. The dry powder was micronized to a particle-size suitable for inhalation therapy (MMD < 3 μm according to image-analysis). A number of gelatine capsules were each filled with 20 mg of the lyophilized and micronized lipid composition. Other gelatine capsules were filled with 20 mg of micronized lactose. The capsules wre used to determine the tolerance of inhaling the lipid-based solid composition in man using a randomized, single blind cross-over study. The powders were administered by inhalation using Spinmatic® inhalator to 10 healthy volunteers. Each volunteer received 3 single doses of the two formulations. There was a wash-out period of at least 2 days between the treatments. No adverse drug experience (ADE) were found in the study.

Example 2

10 g Epikuron® 200 H (Lucas Meyer, Hamburg) was dissolved in 100 g t-butanol under gentle heating. The solution was frozen and lyophlized. 3 g of the resulting powder was dispersed in 270 g of an aqueous solution of lactose (6.67 weight %). Liposomes were formed by heating (maximum temperature 75 °C) the sample for approximately 30 minutes during stirring. The liposome dispersion was flash-frozen by dripping it into liquied nitrogen and was then lyophilized. The dry powder was micronized to a particle-size suitable for inhalation therapy (MMD < 3 μm according to image-analysis). 2 g of the lyophilized and micronized powder was agglomerated to spheres with a diameter of less than 1 mm. This gives a powder with improved flow-proper-ties. The agglomerated powder was filled into powder-in-halers (Turbuhaler®, United States Patent 4,524,769). The powder-inhaler is designed to dispense the agglomerated substance accurately. Inspiration through the Turbuhaler® hence leads to inhalation of a certain amount of the powder.

Example 3

In a 2 l flask 2 g Epikuron® 200 H was dissolved in 20 ml chloroform. The chloroform was evaporated to leave a lipid film on the inner surface of the flask. 110 g of an aqueous solution of lactose (9.1 weight %) was added. Liposomes were formed by shaking the flask at elevated temperature. The liposome-dispersion was spray-dried with a Buchi 190 Mini Spray-Dryer using an inlet temperature of 155 °C. The final traces of water were eliminated by over-night storage under reduced pressure (0.1 torr). The particle size of the spray-dried powder was suitable for inhalation therapy (MMD < 3 μm according to image-analysis). 100 mg of the powder was dispersed in 10 g of a chilled 50:50 w/w mixture of propellant 12 and propellant 114. The mixture was sealed in a glass container fitted with a 50 μl metering valve. The resulting pressurized dose-aerosol was used to produce an aerosol which formed liposomes in an aqueous milieu.

Example 4

1.43 g of a lyophilized Epikuron® 200 H powder (lyophlized from t-butanol as described above) was dispersed in 500 g of an aqueous solution of lactose containing 8.57 g lactose. Liposomes were formed by heating (maximum temperature 70 °C) the sample for 45 minutes during stirring. The liposome dispersion was spray-dried with a Buchi 190 Mini Spray-Dryer using an inlet temperature of 155 °C. The final traces of water were eliminated by over-night storage under reduced pressure (0.1 torr). The particle size of the spray-dried powder was suitable for inhalation therapy (MMD < 3 μm according to image-analysis). 100 mg of the powder was dispersed in 10 g of a chilled 50:50 w/w mixture of propellant 12 and propellant 114. The mixture was sealed in an glass container fitted with a 50 μl metering valve. The resulting pressurized dose-aerosol was used to produce an aerosol which formed liposomes in an aueous milieu.

### Example 5

The procedure of example 4 was repeated except that 3.33 g of lyophilized Epikuron® 200 H powder was dispersed in 500 g of an aqueous solution of lactose containing 6.67 g lactose. The resulting pressurized dose-aerosol was used to produce an aerosol which formed liposomes in an aqueous milieu.

### Example 6

The procedure of example 5 was repeated except that a lyophilized mixture of DPPC and flumethasone-21-palmitate (19:1 w/w/) was used instead of Epikuron® 200 H. The resulting pressurized dose-aerosol was used to produce an aerosol which formed liposomes in an aqueous milieu.

### Example 7

3.04 g DPPC and 0.16 g flumethasone-21-stearate were dissolved in 65 g t-butanol under gentle heating. The solution was frozen and lyophilized. The resulting powde was dispersed in 245 g of an aqueous solution of lactose (6.67 weight %). Liposomes were formed by heating (maximum temperature 70 °C) the sample for at least 30 minutes during stirring. The liposome dispersion was flash-frozen by dripping it into liquid nitrogen and was then lyophilized. The dry powder was micronized to a particle-size suitable for inhalation therapy (MMD < 3 μm according to image-analysis). 5.0 g of the lyophilized and micronized powder was dispersed in 340 g of a chilled 50:50 w/w mixture of propellant 12 and propellant 114. Containers were filled with approximately 10 g each of the suspension and sealed. Both Al-containers and glass containers were used. 50 μl valves with steam of stainless steel, 50 μl valves with steam of plastic and non-metering valves were used to seal the containers. The resulting pressurized dose-aerosols were used to produce an aerosol which formed liposomes in an aqueous milieu.

### Example 8

17.1 g DPPC and 0.9 g flumethasone-21-palmitate were dissolved in 360 g t-butanol under gentle heating. The solution was frozen and lyophilized. 9.5 g of the dry lyophilized powder was dispersed in 812 g of an aqueous solution of lactose (6.67 weight %). Liposomes were formed by heating (maximum temperature 70 °C) the sample for approximately 1 hour during stirring. The liposome dispersion was flash-frozen by dripping it into liquid nitrogen and was then lyophlized. The dry powder was micronized to a particle-size suitable for inhalation therapy (MMD < 3 μm according to image-analysis). 16.3 g of the lyophilized and micronized powder was dispersed in 1100 g of a chilled 50:50 w/w mixture of propellant 12 and propellant 114. Containers were filled with approximately 8.7 g each of the suspension and sealed. Both Al-containers and glass containers were used. 50 μl valves with steam of stainless steel, 50 μl valves with steam of plastic and non-metering valves were used to seal the containers. The resulting pressurized dose-aerosols were used to produce an aerosol which formed liposomes in an aqueous milieu.

### Example 9

16. g of the lyophilized and micronized powder manufactured in example 8 was dispersed in 1100 g of a chilled 65:35 w/w mixture of propellant 114 and propellant 115. Containers were filled with approximately 8.7 g each of the suspension and sealed. Both Al-containers and glass containers were used. 50 μl valves with steam of stainless steel, 50 μl valves with steam of plastic and non-metering valves were used to seal the containers. The resulting pressurized dose-aerosols were used to produce an aerosol which formed liposomes in an aqueous milieu.

### Example 10

The procedure of Example 8 was repeated except that 7.5 g of the dry powder resulting from the lyophilization of the t-butanol solution in that example was dispersed in 314 g of aqueous solution of lactose (4.78 weight %). Furthermore 7 g of the lyophilized and micronized powder was dispersed in the propellant mixture. The resulting pressurized dose-aerosols were used to produce an aerosol which formed liposomes in an aqueous milieu.

### Example 11

6.0 g of the lyophilized and micronized powder manufactured in example 10 was dispersed in 1100 g of a chilled 65:35 w/w mixture of propellant 114 and propellant 115. Containers were filled with approximately 8.7 g each of the suspension and sealed. Both Al-containers and glass containers were used. 50μl valves with steam of stainless steel, 50 μl valves with steam of plastic and non-metering valves were used to seal the containers. The resulting pressurized dose-aerosols were used to produce an aerosol which formed liposomes in an aqueous milieu.

### Example 12

10 parts of DPPC and 1 part of budesonide-21-palmitate were dissolved in 500 parts of t-butanol under gentle heating. The solution was frozen and lyophilized. 1.38 g of the resulting powder was dispersed in 168 g of aqueous solution of lactose (10.0 weight-%). Liposomes were formed by heating (maximum temperature 60 °C) the sample for approximately 30 minutes during stirring. The liposome dispersion was flash-frozen by dripping it into liquid nitrogen and was then lyophilized during three days. The dry powder was micronized to a particle-size suitable for inhalation therapy (MMD $<$ 3 $\mu$m according to image-analysis). 4.35 g of the lyophilized and micronized powder was dispersed in 500 g of a chilled 50:50 w/w mixture of propellant 12 and propellant 114. Containers were filled with approximately 9 g each of the suspension and sealed. Both Al-containers and glass containers were used. 50 $\mu$l valves with steam of stainless steel, 50 $\mu$l valves with steam of plastic and non-metering valves were used to seal the containers. The resulting pressurized dose-aerosols were used to produce an aerosol which formed liposomes in an aqueous milieu.

Example 13

5 g Epikuron® 200 H was dissolved in 135 g t-butanol under gentle heating. The solution was frozen and lyophilized. 4 g of the dry lyophilized powder was dispersed in 200 g distilled water. Liposomes were formed by heating (maximum temperature 70 °C) the sample for 1 hour during stirring. 2 g terbutaline sulphate was dissolved in 100 g distilled water. The both preparations were mixed, frozen and lyo philized. 5 g of the fried product was dispersed in 27 ml distilled water. Liposomes were formed by heating (maximum temperature 70 °C) the samples for 30 minutes under stirring. The dispersion was diluted with 350 g of an aqueous solution of lactose (6.67 weight %). The liposome dispersion was flash-frozen by dripping it into liquid nitrogen and was then lyophilized. The dry powder was micronized to a particle-size suitable fo rinhalation therapy (MMD $<$ 3 $\mu$m according to image-analysis). The encapsulation of terbutaline sulphate into the liposomes was measured spectrophotometically at 280 nm. It was found to be 33 % both before and after micronization. 4.8 g of the lyophilized and micronized powder was dispersed in 556 g of a chilled 50:50 w/w mixture of propellant 12 and propellant 114. Al-containers were filled with approximately 14 g of the suspension and sealed with metering valves. The resulting pressurized dose-aerosols were used to produce an aerosol which formed liposomes in an aqueous milieu.

Example 14

100 mg DMPC dissolved in 1 ml chloroform was added to a flask. The chloroform was evaporated to leave a thin lipid film on the inner surface of the flask. 4 ml distilled water was added. Liposomes were formed by shaking the flask at 35 °C. 100 mg of a protein (catalase) dissolved in 1 ml distilled water was added. The sample was frozen and lyophilized. The resulting powder was dispersed in 1 ml distilled water at 35 °C. When liposomes had formed the sample was diluted with 5 ml of an aqueous solution of lactose containing 500 mg lactose. The liposome dispersion was flash-frozen by drippin git into liquid nitrogen and was then lyophilized. The encapsulation of catalase into the liposomes was determined spectrophotometically at 280 nm to 25 %. 100 mg of the powder was dispersed in 10 g of a chilled 50:50 w/w mixture of propellant 12 and propellant 114. The mixture was sealed in a glass container fitted with a 50 $\mu$l metering valve. The resulting pressurized dose-aerosol was used to produce an aerosol which formed liposomes in an aqueous milieu.

Example 15

The procedure of example 14 was repeated except that DPPC was used instead of DMPC and a xantine (caffeine) was used instead of catalase. Furthermore, the temperature was 60 °C during formation of the liposomes. The resulting pressurized dose-aerosol was used to produce an aerosol which formed liposomes in an aqueous milieu.

Example 16

40 mg DMPC was dissolved in 5 g t-butanol under gentle heating. The solution was frozen and lyophilized. The resulting powder was dispersed in 15 g of a chiled 50:50 w/w mixture of propellant 12 and propellant 114. The mixture was sealed in a glass container fitted with a 50 $\mu$l metering valve. The resulting pressurized dose-aerosol was used to produce an aerosol which formed liposomes in an aqueous milieu.

Example 17

40 mg DMPC and 2 mg flumethasone-21-palmitate were dissolved in 5 g t-butanol under gentle heating. The solution was frozen and lyophilized. The resulting powder was dispersed in 15 g of a chilled 50:50 w/w mixture of propellant 12 and propellant 114. The mixture was sealed in a glass container fitted with a 50 $\mu$l metering valve. The resulting pressurized dose-aerosol was used to produce an aerosol which formed liposomes in an aqueous milieu.

Example 18

7.22 g DPPC and 0.38 g flumethasone-21-palmitate were dissolved in 76 g t-butanol under gentle heating. The solution was frozen and lyophilized. The resulting powder was dispersed in 432 g of an aqueous solution of lactose (3.33 weight %). Liposomes were formed by heating (maximum temperature 65 °C) the sample for at least 30 minutes during stirring. The liposome dispersion was spray-dried with a Buchi 190 Mini Spray-Dryer using an inlet temperature of 155 °C. The particle size of the spray-dried powder was suitable for inhalation therapy (MMD $<$ 3 $\mu$m according to image-analysis). 2.8 g of the lyophilized and micronized powder was dispersed in 434 g of a chilled 65:35 w/w mixture of propellant 114 and propellant 115. Al-containers were filled with approximately 16.7 g each of the suspension and sealed with 50 $\mu$l valves.

The resulting pressurized dose-aerosols were used to produce an aerosol which groups of Sprague Dawley rats were exposed to nose-only in a Battelle inhalation chamber. After the first exposure, the animals were given Sephadex beeds by intratracheal instillation. The animals were then exposed to the aerosol daily for three consecutive days. On the fifth day the animals were sacrificed and the lung weights were examined.

Animals given Sephadex beeds and treatd with placebo pressurized dose-aerosols (containing the same components as described above except for the spray-dried powder) developed pulmonary edema measured as increased lung weight. Groups of animals treated with three different doses from the pressurized dose-aerosols showed a significant dose- response relationship. The high dose level inhibited the development of lung edema and the animals showed the same lung weight as the normal rats in the control group.

**Claims**

1) A system for administration of a lipid-based material to mammals, including man characterized in that it comprises a dry lipid-based solid material in a particulate form, which forms or reconstitutes liposomes in the presence of water, and a device suitable to administer said solid material as an aerosol.

2) A system as claimed in claim 1 wherein the main lipid component of the solid material is one or more phospholipids, eg phosphatidylcholine optionally together with one or more other lipid components.

3) A system as claimed in any one of claims 1 to 2 wherein the solid material contains a sterol and/or a substance which donates a positive or a negative charge to the liposomes.

4) A system as claimed in any one of claims 1 to 3 wherein the solid material contains a pharmacologically active substance or derivatives thereof.

5) A system as claimed in claim 4 wherein the pharmacologically active substance or derivative thereof is entrapped within the liposomes which are formed or reconstituted from the dry lipid-based solid material or partioned between the liposomes, which are formed or reconstituted from the dry lipid-based solid material, and an external phase.

6) A system as claimed in any one of claims 4 to 5, wherein the pharmacologically active substance is a $\beta_2$-re-ceptor active substance, preferably selected from the following substances: terbutaline, salbutamol, mabuterol, fenoterol, formoterol, orciprenaline, isoprenaline, isoetharine, clenbuterol, hexoprenaline, procaterol, 1-(4-hydroxyphenyl)-2-[1,1-dimethyl-3-(2-methoxyphenyl) propylamino]-ethanol, 1-(3,5-dihydroxy--phenyl)-2-[1,1-dimethyl-3-(2-methoxyphenyl) propylamino]-ethanol, 1-3,4-dihydroxyphenyl)-2-[1,1-dimethyl-3-(2-methoxyphenyl) propylamino]-ethanol, (4,hydroxy-$\alpha'$-[[6-(4-phe-nylbutoxy)-hexyl]-amino]-methyl]-1,3 benzyldimethanol, or a pharmacologically acceptable salt or derivative of any one thereof and mixtures thereof.

7) A system as claimed in claim 6 wherein the $\beta_2$-receptor active substance is terbutaline sulphate.

8) A system as claimed in any one of claims 4 to 5 wherein the pharmacologically active substance is a glucocorticosteroid, preferably selected form the following substances: budesonide, beclomethasone, dexamethasone, flumethasone, triamcinolone acetonide, flunisolide, 17-and/or 21-esters of these steroids or a pharmacologically acceptable salt of any one thereof and mixtures thereof.

9) A system as claimed in claim 8 wherein the glucocortiscosteroid is a 21-fatty acid ester of budesonide or flumethasone.

10) A system as claimed in any one of claims 4 to 5, wherein the pharmacologically active substance is a xanthine such as theophylline or enprofylline, or cromoglycate, lidocaine, indometacin, diclofenac, ibuprofen, piroxicam, sulindac, or a pharmacologically acceptable salt or derivative of any one thereof and mixtures thereof.

11) A system as claimed in any one of claims 1 to 10 wherein the solid material contains a filler material, preferably a carbohydrate, eg lactose, trehalose or sucrose.

12) A system as claimed in any one of claims 1 to 11 wherein the solid material is manufactured by dehydration of a liposome dispersion preferably by lyophilization or spray-drying.

13) A system as claimed in any one of claims 1 to 12 wherein the solid material is administered by any of the following routes; oral, rectal, vaginal, in open wounds, or by inhalation.

14) A system as claimed in any one of claims 1 to 13, wherein the solid material has a particle size such as at least 95 % by weight is less than 10 microns in diameter.

15) A system as claimed in any one of claims 1 to 14, wherein the solid material is administered by means of a powder-inhalator.

16) A system as claimed in any one of claims 1 to 14, wherein the solid material is administered by means of a self-propelling powder-dispensing composition.

17) A system as claimed in claim 16 wherein the self-propelling powder dispensing aerosol composition comprises a liquefied propellant in which said solid material is substantially insoluble, preferably a hologenated hydrocarbon eg
propellant 12 ($CCl_2F_2$)
propellant 114 ($CClF_2CClF_2$)
propellant 114 ($CClF_2CF_3$)
or propellant C-318 ($C_4F_8$)
or a mixture of halogenated hydrocarbons.

18) A package comprising a pressure-tight container having a valve-controlled opening and containing a solid material and a self-propelling powder-dispensing composition as claimed in any one of claim 16 to 17.

19) A pharmaceutical package as claimed in claim 18 capable of providing a measured dose of medicament in aerosol form suitable for inhalation therapy.

20) Use of a system according to any of claims 1 to 17 or a package according to any of claims 18 to 19 for administration of lipid-based material to mammals, including man.

European. Patent
Office

**PARTIAL EUROPEAN SEARCH REPORT**
which under Rule 45 of the European Patent Convention
shall be considered, for the purposes of subsequent
proceedings, as the European search report

Application number

87850273.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | EP-A-158 441 (PHARES PHARMACEUTICAL RESERACH NV) <br> * see claims 1-12, 14, page 7, lines 31-37, page 8, lines 5-21, examples * <br> & JP, 61044808 <br> --- | 1-6, 8, 10, 11, 13-16 | A 61 K 9/12, 9/72, 47/00, 31/685 |
| Y | SE-A-8404118-5 (STERWIN AG) <br> * see claim 13 examples 5-7 * <br> & GB, A, 2145107 <br> --- | 1-4, 13, 15-19 | |
| Y | GB-A-1 575 343 (IMPERIAL CHEMICAL INDUSTRIES LTD) <br> * see claims, page 2, lines 72-91, examples * <br> & NL, 7805005  SE, 8201351 <br> BE, 866697  US, 4370349 <br> FR, 2390159  SE, 440725 <br> DE, 2818655 <br> JP, 53142514 <br> US, 4311712 <br> AU, 514644 <br> CA, 1114758 <br> SE, 8201350  ___   -/- | 1-4, 12 | **TECHNICAL FIELDS SEARCHED (Int. Cl.4)** <br><br> A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely:

Claims not searched: 20

Reason for the limitation of the search:

Method for the treatment of the human or animal body
/EPC Art. 52 (4)7.

| Place of search | Date of completion of the search | Examiner |
|---|---|---|
| STOCKHOLM | 03-12-1987 | TANNERFELDT A. |

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| Y | GB-A-1 575 344 (IMPERIAL CHEMICAL INDUSTRIES LTD) <br> * see claims, examples * <br> --- | 1-4, 12 | |
| D | WO-A1-86/06959 (LIPOSOME TECHNOLOGY INC) <br> * see claims, page 4, line 5 - <br> - page 6, line 19 * <br> --- | 1-8, 10, 12-14, 17-19 | |
| | | | TECHNICAL FIELDS SEARCHED (Int. Cl.4) |

EPO Form 1505.3 06.78